# EUROPÄISCHE PATENTSCHRIFT

(11) **EP 0 569 609 B1**
(45) Veröffentlichungstag und Bekanntmachung des Hinweises auf die Patenterteilung: **10.01.2001**
(21) Anmeldenummer: 92107996.8
(22) Anmeldetag: 12.05.1992
(51) Int. Cl.: A61N 1/39, A61N 1/08

(54) **Implantierbares Defibrillationssystem**
Implantable defibrillator system
Système de défibrillateur implantable

(43) Veröffentlichungstag der Anmeldung: 18.11.1993
(73) Patentinhaber: Pacesetter AB, 171 95 Solna (SE)
(72) Erfinder: Hirschberg, Jakub, S-183 44 Täby (SE); Strandberg, Hans, S-172 36 Sundbyberg (SE)
(74) Vertreter: Lettström, Richard Wilhelm

(56) Entgegenhaltungen:
- EP-A- 0 060 404
- EP-A- 0 228 539
- DE-A- 3 800 018
- FR-A- 2 653 023
- US-A- 4 320 763
- US-A- 4 800 883
- DATABASE WPIL Week 9123, Derwent Publications Ltd., London, GB; AN 91-169078

## Beschreibung

Die Erfindung betrifft ein implantierbares Defibrillationssystem bestehend aus einem implantierbaren Defibrillator und aus Elektrodenanschlüsse zum anschliessen von Elektrodenzuleitungen, der Defibrillator bestehend aus einer Ladekapazität, einer Ladeschaltung und einer Schalteinrichtung, wobei die Ladekapazität an ihren beiden Seiten zum Aufladen an die Ladeschaltung schaltbar ist und zur Entladung um Defibrillieren eines Herzens über die Schalteinrichtung und die Elektrodenanschlüsse mit mindestens zwei, im Bereich des Herzens angeordneten Elektroden verbindbar ist.

Ein derartiger, aus der US-A-4 800 883 bekannter Defibrillator ist zur Implantation in dem Körper eines Patienten vorgesehen. Der bekannte Defibrillator enthält eine Ladekapazität bestehend aus zwei Kondensatoren, die zur Aufladung auf eine vorgegebene Spannung an einer Ladeschaltung angeschlossen sind. Die beiden Kondensatoren sind ferner über eine Schalteinrichtung bestehend aus vier, in einer Brückenschaltung angeordneten Schaltern mit zwei am Herzen des Patienten plazierten Elektroden verbunden. Zur Defibrillation des Herzens wird die Ladekapazität zuerst auf die vorgegebene Spannung aufgeladen und anschließend über die Schalteinrichtung mit den Elektroden am Herzen verbunden, so daß sich die Ladekapazität mit einem Entladestrom über das Herzgewebe entlädt. Indem die vier Schalter der Schalteinrichtung abwechselnd paarweise geöffnet und geschlossen werden, wird der Entladestrom durch das Herzgewebe in eine Mehrzahl von aufeinanderfolgenden Teilströmen mit alternierdender stromrichtung aufgeteilt.

Der die Defibrillierung zustande bringende Strom durch das Herzgewebe ist von der Ladespannung der Ladekapazität und dem elektrischen Widerstand des Herzgewebes zwischen den Elektroden abhängig. Zu Beginn der Entladung der Ladekapazität weist der Strom seinen höchsten Wert auf, um dann exponentiell abzuklingen. Um eine effektive Defibrillation des Herzens zu erhalten, muß der Strom durch das Herzgewebe während einer bestimmten Dauer einen bestimmten Mindestwert überschreiten. Aus diesem Grund wird die Ladespannung für die Ladekapazität so gewählt, daß der Strom zu Beginn des Entladevorgangs ausreichend groß ist, um erst nach Ablauf der bestimmten Dauer unter den Mindestwert abzusinken. Der den Mindestwert übersteigende Anteil des Stromes ist im Hinblick auf eine effektive Defibrillation nicht nur ohne Nutzen, sondern kann darüberhinaus auch auf Grund des hohen Anfangswertes zu Schädigungen des Herzgewebes führen. Ein weiteres Problem besteht darin, daß es auf Grund eines Fehlers oder Schadens in den Zuleitungen der Elektroden oder bei Verlagerung der Elektroden zwischen diesen zu Kurzschlüssen kommen kann, so daß der bei Auslösung der Entladung der Ladekapazität entstehende Kurzschlußstrom Schädigungen des Patienten und des Defibrillators verursachen kann. Schließlich können derartige Kurzschlüsse auch bei der Herstellung und beim späteren Hantieren des Defibrillators im Zusammenhang mit seiner Implantation auftreten, so daß eine Gefährdung der den Defibrillator hantierenden Person besteht.

In der US-A-4 320 763 wird ein Pacemaker beschrieben, der durch einen mit Pulsgenerator und Elektrode in Reihe geschalteten strombegrenzenden Kreis gegen äussere hohe Spannungen, z.B. von einem äusseren Defibrillator herrührend, geschützt wird. Der strombegrenzende Kreis kann beispielsweise eine strombegrenzende Diode und ein in Reihe geschaltetes Paar Feldleistungstransistoren umfassen. Durch diese Einrichtung wird auch das Gewebe vor den starken Strömen geschützt, die von dem Kreis des Pacemakers durch dessen im Vergleich zum umgebenden Gewebe höhere Leitfähigkeit aufgefangen werden können.

In der EP-A1-0 228 539 wird eine Schutzvorrichtung beschrieben, die einen Pacemaker vor allzu hohen Strömen, die entstehen können, wenn eine Defibrillator zur Defibrillierung des Herzens zusammen mit dem Pacemaker verwendet wird, schützen soll. Insbesondere soll die Schutzvorrichtung mit einem Pacemaker, der in einer gewissen Ausdehnung einen gemeinsamen Rückleiter mit einen Defibrillator teilen kann, in Reihe geschaltet werden.

Der Erfindung liegt daher die Aufgabe zugrunde, bei einem Defibrillator die Entstehung von allzu hohen Entladeströmen zu verhindern, ohne daß jedoch dadurch die Effektivität der Defibrillierung beeinflußt wird.

Gemäß der Erfindung wird die Aufgabe dadurch gelöst, daß bei dem Defibrillationssystem der eingangs angegebenen Art in dem Stromweg von der Ladekapazität zu den Elektrodenanschlüssen eine Einrichtung zur Begrenzung des Stromes auf einen vorgegebenen Maximalwert, unabhängig von der Ladespannung der Ladekapazität und dem elektrischen Widerstand des Herzgewebes zwischen den Elektroden, angeordnet ist. Dadurch wird erreicht, daß beim normalen Betrieb des Defibrillators der Entladestrom der Ladekapazität und damit der Strom durch das Herzgewebe auf den vorgegebenen Maximalwert begrenzt ist, so daß eine Schädigung des Herzgewebes ausgeschlossen ist. Dieselbe Strombegrenzung ist auch im Falle einer Störung beispielsweise eines Kurzschlusses zwischen den Elektroden wirksam, so daß eine Schädigung des Defibrillators verhindert wird.

Eine konstruktiv besonders einfache und darüber hinaus platzsparende Ausbildung der Einrichtung zur Strombegrenzung wird dadurch erreicht, daß diese aus einem Widerstandselement mit positiver Temperaturcharakteristik (PTC-Widerstand) besteht. Ein solches Widerstandselement, das überlicherweise aus einer halbleitenden Keramik besteht, erhöht seinen Widerstandswert bei zunehmender Temperatur durch den das Widerstandselement durchfließenden Strom, wodurch der Strom begrenzt wird.

Es sind seit einiger Zeit Widerstandselemente aus halbleitendem Polymer bekannt, die als selbstheilende Sicherungen wirken, indem sie bei einem bestimmten Temperaturwert, der einem bestimmten Strom entspricht, ihren Widersstandswert schlagartig erhöhen und so eine Stromunterbrechung bewirken, bis die Temperatur unter den vorgegebenen Wert abgesunken ist. Ist bei dem erfindungsgemäßen Defibrillationssystem nur ein Schutz gegen eventuelle Kurzschlüsse gewünscht, so ist ein derartiges Widerstandselement wegen seiner schnellen Ansprechgeschwindigkeit von besonderem Vorteil.

Entsprechend einer alternativen Ausbildung des erfindungsgemäßen implantierbaren Defibrillationssystem ist vorgesehen, daß die Einrichtung zur Strombegrenzung einen in dem Stromweg angeordneten niederohmigen Meßwiderstand aufweist, daß parallel zu dem Meßwiderstand eine den durch den Strom im Meßwiderstand verursachten Spannungsabfall erfassende Meßschaltung liegt und daß die Meßschaltung ausgangsseitig an dem Steuereingang eines steuerbaren Widerstands oder Schalters angeschlossen ist, der entweder in Reihe mit dem Meßwiderstand in dem Stromweg angeordnet ist und beim Erreichen des Maximalwertes für den Strom in einen höherohmigen bzw. offenen Zustand gesteuert wird, oder in einem Stromzweig parallel zu der Ladekapazität angeordnet ist und beim Erreichen des Maximalwertes für den Strom in einen niedrigerohmigen bzw. geschlossenen Zustand gesteuert wird. Dabei erfolgt die Strombegrenzung unabhängig von irgendwelchen Temperaturausgleichsvorgängen und ist deswegen besonders schnell und genau. Darüber hinaus besteht die Möglichkeit, durch Veränderung des Steuerverhaltens der Meßschaltung unterschiedliche Werte für den Maximalwert des zu begrenzenden Stromes einzustellen.

Als besonders vorteilhaft erweist sich die gemäß der Erfindung vorgesehene Strombegrenzung, wenn der implantierbare Defibrillator ein implantierbares Gehäuse umfasst, indem die Ladeschaltung, die Ladekapazität und die Schalteinrichtung angeordnet sind. Da nach einer Impantation eines derartigen Defibrillators die Elektrodenzuleitungen und Elektroden nicht mehr ohne weiteres zugänglich sind, besteht ein besonders großer Bedarf nach einer Strombegrenzung für den Fall, daß die Elektrodenleitungen oder Elektroden im Körper des Patienten ihre Lage ändern und so Kurzschlüsse verursachen können. Dabei ist die Einrichtung zur Strombegrenzung vorzugsweise in dem Gehäuse angeordnet, so daß die Strombegrenzung unabhängig davon erfolgt, an welcher Stelle der Elektrodenleitungen ein möglicher Kurzschluß auftritt. Es besteht aber auch die Möglichkeit, daß das implantierbare Defibrillationssystem eine Elektrodenzuleitung enthält, in der, bei entsprechender Isolierung der Elektrodenzuleitung, die Einrichtung zur Strombegrenzung angeordnet ist.

Um auch herkömmliche Defibrillatoren ohne Mittel zur Strombegrenzung entsprechend nachrüsten zu können, ist in vorteilhafter Weise vorgesehen, daß die Einrichtung zur Strombegrenzung in einem Steckerteil angeordnet ist, das mit Anschlußelementen zur Verbindung mit dem Gehäuse des Defibrillators und mit weiteren Anschlußelementen zum Anschließen zumindest einer der Elektroden versehen ist.

Zur Erläuterung der Erfindung wird im folgenden auf die Figuren der Zeichnung Bezug genommen. Im einzelnen zeigen
- FIG. 1: ein vereinfachtes Blockschaltbild eines Defibrillators mit einem PTC-Widerstand zur Strombegrenzung,
- FIG. 2: ein Beispiel für die Anordnung des PTC-Widerstands in einem mit dem Gehäuse des Defibrillators verbindbaren Steckerteil,
- FIG. 3: ein vereinfachtes Blockschaltbild einer alternativen Ausbildung der Einrichtung zur Strombegrenzung mit einem in Abhängigkeit von dem Strom durch einen Meßwiderstand gesteuerten steuerbaren Widerstand,
- FIG. 4: in einem Diagramm den Ausgangsstrom des Defibrillators mit und ohne Strombegrenzung,
- FIG. 5: ein Beispiel für die schaltungstechnische Ausführung der Strombegrenzung nach Fig. 3 und
- FIG. 6: eine alternative Ausbildung der Einrichtung zur Strombegrenzung mit einem den überschüssigen Stromanteil von dem zu begrenzenden Strom abzweigenden steuerbaren Widerstand.

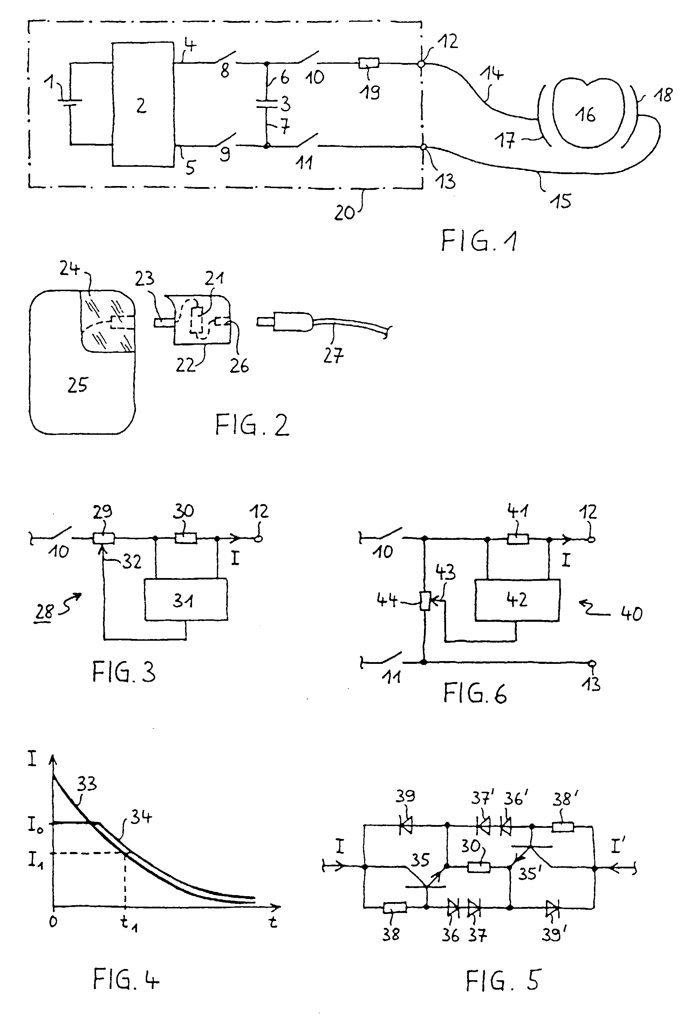

Der in Figur 1 als Blockschaltbild dargestellte Defibrillator enthält eine Spannungsquelle 1 in Form einer Batterie, die an einer Ladeschaltung 2 für eine Ladekapazität 3 angeschlossen ist. Die Ladeschaltung 2 erzeugt an ihren Ausgangsanschlüssen 4 und 5 eine vorgegebene Ladespannung, auf die die Ladekapazität 3 aufgeladen wird, wenn sie an ihren beiden Seiten 6 und 7 über zwei steuerbare Schalter 8 und 9 mit den Ausgangsanschlüssen 4 und 5 verbunden wird. Die beiden Seiten 6 und 7 der Ladekapazität 3 sind ferner über eine steuerbare Schalteinrichtung bestehend aus zwei weiteren Schaltern 10 und 11 mit jeweils zwei Elektrodenanschlüssen 12 und 13 verbindbar, an denen über jeweils eine Elektrodenleitung 14 bzw. 15 zwei im Bereich des Herzens 16 eines Patienten angeordnete Elektroden 17 und 18 angeschlossen sind. In dem Stromweg von der Ladekapazität 3 zu den Elektroden 17 und 18 ist zwischen dem steuerbaren Schalter 10 und dem Elektrodenanschluß 12 ein Widerstandselement 19 mit positiver Temperaturcharakteristik (PTC) angeordnet. Das Widerstandselement 19, daß aus einer halbleitenden Keramik oder einem halbleitenden Polymer besteht, weist bei einem Strom unterhalb eines vorgegebenen Maximalwertes einen niedrigen Widerstandswert auf. Wenn der elektrische Strom durch das Widerstandselement 19 den vorgegebenen Maximalwert erreicht, steigt der Widerstandswert des Widerstandselements 19 auf Grund des Temperaturanstiegs in dem Widerstandselement 19 markant an und begrenzt bzw. unterbricht im Falle des halbleitenden Polymers auf diese Weise den Strom durch das Widerstandselement 19. Damit ist sichergestellt, daß im Falle eines Kurzschlusses zwischen den Elektrodenleitungen 14 und 15 oder den Elektroden 17 und 18 der Entladestrom der Entladungskapazität 3 auf den vorgegebenen Maximalwert begrenzt ist.

Das in Figur 1 gezeigte Ausführungsbeispiel zeigt nur die zum Verständnis der Erfindung wesentlichen Teile des Defibrillators. So sind insbesondere nicht die Schaltungsteile dargestellt, die zur Steuerung der Schalter 8 bis 11 sowie zur Steuerung der Ladeschaltung 2 zwecks Einstellung der vorgegebenen Ladespannung für die Ladekapazität 3 erforderlich sind. Ferner kann die Ladekapazität 3 aus mehreren Kondensatoren bestehen und die steuerbare Schalteinrichtung 10,11 ebenso wie bei dem aus der eingangs genannten US-A-4 800 883 bekannten Defibrillator als Brückenschaltung ausgebildet sein, so daß der Entladestrom der Ladekapazität 3 in entgegengesetzter Richtung das Herz 16 durchfließen kann. Die Elektroden 17 und 18 können wie gezeigt als Flächenelektroden oder als Katheterelektroden ausgebildet sein und außerhalb des Herzens 16 oder in diesem angeordnet sein. Auch können weitere Elektroden vorgesehen werden, die mit den gezeigten Elektroden 17 und 18 verbunden sind.

Bei dem in Figur 1 gezeigten Ausführungsbeispiel sind die Batterie 1, die Ladeschaltung 2, die Ladekapazität 3, die steuerbaren Schalter 8 bis 11 und das strombegrenzende Widerstandselement 19 in einem zur Implantation in den Körper des Patienten vorgesehenen Gehäuse 20 untergebracht.

Figur 2 zeigt eine dazu alternative Anordnung einer strombegrenzenden Einrichtung 21 in einem Steckerteil 22, das einen Anschlußstift 23 zum Verbinden des Steckerteils 22 mit dem Anschlußteil 24 eines implantierbaren Defibrillators 25 aufweist und eine Anschlußbuchse 26 zum Anschliessen einer Elektrodenzuleitung 27 enthält. Damit besteht die Möglichkeit, bisher bekannte Defibrillatoren ohne Strombegrenzung entsprechend nachzurüsten.

Figur 3 zeigt eine alternative Ausbildung für die Einrichtung zur Strombegrenzung 28, wobei im Stromweg von der Ladekapazität zu den Elektroden - bezogen auf Figur 1 also zwischen dem steuerbaren Schalter 10 und dem Elektrodenanschluß 12 - ein steuerbarer Widerstand 29 in Reihenschaltung mit einem niederohmigen Meßwiderstand 30 angeordnet ist. Eine Meßschaltung 31 ist mit ihrem Meßeingang parallel zu dem Meßwiderstand 30 angeordnet und ausgangsseitig mit einem Steuereingang 32 des steuerbaren Widerstands 29 verbunden. Die Meßschaltung 31 erfaßt an ihrem Meßeingang den von dem Strom I in dem Meßwiderstand 30 hervorgerufenen Spannungsabfall und steuert den steuerbaren Widerstand 29 in der Weise, daß beim Erreichen eines vorgegebenen Maximalwertes I₀ für den Strom I der Widerstand 29 in einen hochohmigen Zustand gesteuert wird, so daß der Strom I auf den Maximalwert I₀ begrenzt wird. Anstelle des steuerbaren Widerstands 29 ist auch ein steuerbarer Schalter denkbar, der jedesmal dann, wenn der Strom I den Maximalwert I₀ erreicht, eine Unterbrechung des Stromflusses bewirkt.

Figur 4 verdeutlicht dies an Hand zweier Stromverläufe 33 und 34, wovon der mit 33 bezeichnete Stromverlauf eintritt, wenn zum Zeitpunkt t=0 die Schalter 10 und 11 geschlossen werden und sich die Ladekapazität 3 ohne irgendwelche Strombegrenzung über das Herzgewebe 16 entlädt. Der Stromverlauf 33 weist zu Beginn der Entladung der Ladekapazität 3 seinen höchsten Wert auf und klingt danach exponentiell ab, wobei zum Zeitpunkt t₁ die mit I₁ bezeichnete Mindeststromstärke zur effektiven Defibrillation des Herzens 16 unterschritten wird. Wie Figur 4 zeigt, kann der Anfangswert des Stromverlaufs 33 erheblich über der Defibrillationsschwelle I₀ liegen, wobei Schädigungen des Herzgewebes 16 nicht auszuschließen sind. Demgegenüber erhält man mit der Einrichtung zur Strombegrenzung 28, wie sie in Figur 3 gezeigt ist, den mit 34 bezeichneten Stromverlauf, bei dem der Strom I am Anfang der Entladung der Ladekapazität auf einen unschädlichen Maximalwert I₀ begrenzt wird und zusätzlich in vorteilhafter Weise über den Zeitpunkt t₁ hinaus über dem zur Defibrillation erforderlichen Mindeswert I₁ liegt.

Figur 5 zeigt eine schaltungstechnische Ausführungsmöglichkeit der in Figur 3 nur schematisch gezeigten Einrichtung zur Strombegrenzung 28. Dabei ist der steuerbare Widerstand 29 in Form eines Transistors 35 ausgebildet, der emitterseitig in Reihe mit dem Meßwiderstand 30 angeordnet ist. Zwischen der Basis des Transistors 35 und dem emitterfernen Anschluß des Meßwiderstands 30 liegen zwei Dioden 36 und 37 in Reihe, die von einem mittels eines Vorwiderstandes 38 zwischen dem Kollektor und der Basis des Transistors 35 von dem Strom I abgezweigten Teilstrom gespeist werden. Auf diese Weise wird der Strom I auf den Maximalwert I₀ = (2U_{D}-U_{BE})/R begrenzt, wobei U_{D} die Durchlaßspannung jeweils einer der Dioden 36 und 37, U_{BE} die Basis-Emitter-Spannung des Transistors 35 und R der Widerstandswert des Meßwiderstandes 30 ist. Um auch bei einem entgegengesetzt zu dem Strom I fließenden Strom I' eine Strombegrenzung zu erhalten, ist ein weiterer Transistor 35' mit Dioden 36' und 37' und einem Vorwiderstand 38' in spiegelbildlicher Anordnung bezogen auf den Meßwiderstand 30 vorgesehen, wobei zwei weitere Dioden 39 und 39' in antiparalleler Anordnung zu den Kollektor - Emitter - Strecken der Transistoren 35 und 35' den gegen die Durchlaßrichtung des jeweiligen Transistors 35 bzw. 35' fließenden Strom I' bzw. I an diesem vorbeileiten.

Figur 6 zeigt schließlich ein Ausführungsbeispiel für die strombegrenzende Einrichtung 40, bei dem im Stromweg von der Ladekapazität zu den Elektroden - bezogen auf Figur 1 also zwischen dem steuerbaren Schalter 10 und dem Elektrodenanschluß 12 - ein steuerbarer Meßwiderstand 41 angeordnet ist. Eine Meßschaltung 42 ist mit ihrem Meßeingang parallel zu dem Meßwiderstand 41 angeordnet und ausgangsseitig mit einem Steuereingang 43 eines steuerbaren Widerstands 44 verbunden, der zwischen der Verbindungsstelle des Schalters 10 mit dem Meßwiderstand 41 einerseits und dem Elektrodenanschluß 13 andererseits angeordnet ist. Die Meßschaltung 42 erfaßt an ihrem Meßeingang den von dem Strom I in dem Meßwiderstand 41 hervorgerufenen Spannungsabfall und steuert den steuerbaren Widerstand 44 in der Weise, daß beim Erreichen eines vorgegebenen Maximalwertes I₀ für den Strom I der Widerstand 44 in einen niederohmigen Zustand gesteuert wird, so daß von dem Entladestrom aus der Ladekapazität 3 ein Teilstrom über den steuerbaren Widerstand 44 abgeleitet wird und so der Strom I durch das Herzgewebe 16 auf den Maximalwert I₀ begrenzt wird.

### Bezugszeichenliste

- 1: Batterie
- 2: Ladeschaltung
- 3: Ladekapazität
- 4,5: Ausgangsanschlüsse von 2
- 6,7: die beiden Seiten von 3
- 8,9: steuerbare Schalter
- 10,11: weitere Schalter
- 12,13: Elektrodenanschlüsse
- 14,15: Elektrodenleitungen
- 16: Herz
- 17,18: Elektroden
- 19: Widerstandselement
- 20: Gehäuse für 1,2,3,8-11 und 19
- 21: strombegrenzende Einrichtung
- 22: Steckerteil
- 23: Anschlußstift
- 24: Anschlußteil
- 25: Defibrillator
- 26: Anschlußbuchse
- 27: Elektrodenzuleitung
- 28: Einrichtung zur Strombegrenzung
- 29: steuerbarer Widerstand
- 30: Meßwiderstand
- 31: Meßschaltung
- 32: Steuereingang von 29
- 33,34: Stromverläufe für I
- 35,35': Transistoren
- 36,36',37,37': Dioden
- 38,38': Vorwiderstände
- 39,39': weitere Dioden
- 40: strombegrenzende Einrichtung
- 41: Meßwiderstand
- 42: Meßschaltung
- 43: Steuereingang von 44
- 44: steuerbarer Widerstand
- I,I': Strom
- I₀: Maximalwert für I
- I₁: Mindeststromstärke
- t₁: Zeitpunkt

## Patentansprüche

1. Implantierbares Defibrillationssystem bestehend aus einem implantierbaren Defibrillator und aus Elektrodenanschlüsse (12, 13, 26) zum anschliessen von Elektrodenzuleitungen (14, 15, 27), der Defibrillator bestehend aus einer Ladekapazität (3), einer Ladeschaltung (2) und einer Schalteinrichtung (10, 11), wobei die Ladekapazität (3) an ihren beiden Seiten (6, 7) zum Aufladen an die Ladeschaltung (2) schaltbar ist und zur Entladung um Defibrillieren eines Herzens (16) über die Schalteinrichtung (10, 11) und die Elektrodenanschlüsse (12, 13, 26) mit mindestens zwei, im Bereich des Herzens (16) angeordneten Elektroden (17, 18) verbindbar ist, **dadurch gekennzeichnet,** daß in dem Stromweg von der Ladekapazität (3) zu den Elektrodenanschlüssen (12, 13, 26) eine Einrichtung (19, 21, 28, 40) zur Begrenzung des Stromes (I) auf einen vorgegebenen Maximalwert (I₀), unabhängig von der Ladespannung der Ladekapazität und dem elektrischen Widerstand des Herzgewebes zwischen den Elektroden, angeordnet ist.

2. Implantierbares Defibrillationssystem nach Anspruch 1, **dadurch gekennzeichnet,** daß die Einrichtung zur Strombegrenzung aus einem PTC-Widerstandselement (19,21) mit positiver Temperaturcharakteristik besteht.

3. Implantierbares Defibrillationssystem nach Anspruch 1, **dadurch gekennzeichnet,** daß die Einrichtung zur Strombegrenzung aus einem Widerstandselement (19,21) aus einem halbleitenden Polymer besteht, das bei den vorgegebenen Maximalwert (I₀) seinen Widerstandswert schlagartig erhöhen.

4. Implantierbares Defibrillationssystem nach Anspruch 1, **dadurch gekennzeichnet**, daß die Einrichtung zur Strombegrenzung (28,40) einen in dem Stromweg angeordneten niederohmigen Meßwiderstand (30,41) aufweist, daß parallel zu dem Meßwiderstand (30,41) eine den durch den Strom (I) im Meßwiderstand (30,41) verursachten Spannungsabfall erfassende Meßschaltung (31,42) liegt und daß die Meßschaltung (31,42) ausgangsseitig an dem Steuereingang (32,43) eines steuerbaren Widerstands (29,44) oder Schalters angeschlossen ist, der entweder in Reihe mit dem Meßwiderstand (30) in dem Stromweg angeordnet ist und beim Erreichen des Maximalwertes (I₀) für den Strom (I) in einen höherohmigen bzw. offenen Zustand gesteuert wird, oder in einem Stromzweig parallel zu der Ladekapazität (3) angeordnet ist und beim Erreichen des Maximalwertes (I₀) für den Strom in einen niedrigerohmigen bzw. geschlossenen Zustand gesteuert wird.

5. Implantierbares Defibrillationssystem nach einem der vorangehenden Ansprüche **dadurch gekennzeichnet,** daß der implantierbare Defibrillator ein implantierbares Gehäuse (20) umfasst, in dem die Ladeschaltung (2), die Ladekapazität (3) und die Schalteinrichtung (10,11) angeordnet sind.

6. Implantierbares Defibrillationssystem nach Anspruch 5, **dadurch gekennzeichnet,** daß die Einrichtung zur Strombegrenzung (19,28,40) in dem Gehäuse (20) angeordnet ist.

7. Implantierbares Defibrillationssystem nach Anspruch 5, **dadurch gekennzeichnet,** daß das implantierbare Defibrillationssystem eine Elektrodenzuleitung enthält, in der die Einrichtung zur Strombegrenzung angeordnet ist.

8. Implantierbares Defibrillationssystem nach Anspruch 5, **dadurch gekennzeichnet,** daß das implantierbare Defibrillationssystem ein mit Anschlußelementen (23) zur Verbindung mit dem Gehäuse des Defibrillators (25) und mit weiteren Anschlusselementen (26) zum Anschließen zumindest einer der Elektroden versehenes Steckerteil (22) enthält, in dem die Einrichtung (21) zur Strombegrenzung angeordnet ist.

## Claims

1. Implantable defibrillation system consisting of an implantable defibrillator and of electrode connections (12, 13, 26) for connecting electrode leads (14, 15, 27), the defibrillator consisting of a charging capacitor (3), a charging circuit (2) and a switching device (10, 11), wherein the charging capacitor (3) can be switched on both of its sides (6, 7) for charging to the charging circuit (2) and can be connected to at least two electrodes (17, 18), which are disposed in the region of the heart (16), via the switching device (10, 11) and the electrode connections (12, 13, 26) for discharging in order to defibrillate a heart (16), characterised in that a device (19, 21, 28, 40) for limiting the current (I) to a predetermined maximum value (Iₒ), irrespective of the charging voltage of the charging capacitor and the electrical resistance of the heart tissue between the electrodes, is disposed in the current path from the charging capacitor (3) to the electrode connections (12, 13, 26).

2. Implantable defibrillation system according to Claim 1, characterised in that the device for limiting the current consists of a PTC resistor element (19, 21) with a positive temperature characteristic.

3. Implantable defibrillation system according to Claim 1, characterised in that the device for limiting the current consists of a resistor element (19, 21) of a semiconducting polymer, the resistance of which increases abruptly at the predetermined maximum value (Iₒ).

4. Implantable defibrillation system according to Claim 1, characterised in that the device (28, 40) for limiting the current comprises a low-value measuring resistor (30, 41), which is disposed in the current path, that a measuring circuit (31, 42), which detects the voltage drop caused by the current (I) in the measuring resistor (30, 41), lies parallel to the measuring resistor (30, 41), and that the measuring circuit (31, 42) is connected on the output side to the control input (32, 43) of a controllable resistor (29, 44) or switch, which either is disposed in series with the measuring resistor (30) in the current path and is controlled into a higher value or open state when the current (I) reaches the maximum value (Iₒ), or is disposed in a current path parallel to the charging capacitor (3) and is controlled into a lower value or closed state when the current reaches the maximum value (Iₒ).

5. Implantable defibrillation system according to any one of the preceding Claims, characterised in that the implantable defibrillator comprises an implantable housing (20), in which the charging circuit (2), the charging capacitor (3) and the switching device (10, 11) are disposed.

6. Implantable defibrillation system according to Claim 5, characterised in that the device (19, 28, 40) for limiting the current is disposed in the housing (20).

7. Implantable defibrillation system according to Claim 5, characterised in that the implantable defibrillation system includes an electrode lead, in which the device for limiting the current is disposed.

8. Implantable defibrillation system according to Claim 5, characterised in that the implantable defibrillation system includes a male connector part (22), which is provided with connection elements (23) for connection to the housing of the defibrillator (25) and with further connection elements (26) for connecting at least one of the electrodes, and in which the device (21) for limiting the current is disposed.

## Revendications

1. Système de défibrillateur implantable comprenant un défibrillateur implantable et des bornes d'électrodes (12, 13, 26) pour connecter des lignes d'alimentation d'électrodes (14, 15, 27), le défibrillateur étant formé d'une capacité (3), d'un circuit de charge (2) et d'un dispositif de commutation (10, 11), la capacité (3) pouvant être connectée, pour sa charge, au circuit de charge (2), par l'intermédiaire de ses deux bornes (6, 7) et, pour sa décharge aux fins de défribriller un coeur (16), à au moins deux électrodes (17, 18) disposées dans le voisinage du coeur (16), par l'intermédiaire du dispositif de commutation (10, 11) et des bornes d'électrodes (12, 13, 26), caractérisé en ce qu'un dispositif (19, 21, 28, 40) pour limiter le courant (I) à une valeur maximale (I₀), indépendante de la tension de charge de la capacité et de la résistance électrique du tissu cardiaque entre les électrodes, est disposé dans le trajet de courant entre la capacité (3) et les bornes d'électrodes (12, 13, 26).

2. Système de défibrillateur implantable selon la revendication 1, caractérisé en ce que le dispositif de limitation du courant est formé d'un élément résistant CTP (19, 21) à coefficient de température positif.

3. Système de défibrillateur implantable selon la revendication 1, caractérisé en ce que le dispositif de limitation du courant est formé d'un élément résistant (19, 21) en un polymère semi-conducteur dont la résistance augmente brusquement à la valeur maximale (I₀) prédéterminée.

4. Système de défibrillateur implantable selon la revendication 1, caractérisé en ce que le dispositif de limitation du courant (28, 40) comprend une résistance de mesure (30, 41) de faible valeur ohmique disposée dans le trajet de courant, en ce qu'un circuit de mesure (31, 42) qui mesure la chute de tension engendrée par le passage du courant (I) dans la résistance de mesure (30, 41) est connecté en parallèle avec la résistance de mesure (30, 41) et en ce que le circuit de mesure (31, 42), côté sortie, est connecté à l'entrée de commande (32, 43) d'une résistance commandée (29, 44) ou d'un commutateur qui, soit est branché en série avec la résistance de mesure (30) dans le trajet de courant et est commuté vers un état à valeur ohmique plus élevée ou vers l'état ouvert lorsque la valeur maximale (I₀) pour le courant (I) est atteinte, soit est branché dans une branche de circuit en parallèle avec la capacité (3) et est commuté vers un état à valeur ohmique plus faible ou vers l'état fermé, lorsque la valeur maximale (I₀) pour le courant est atteinte.

5. Système de défibrillateur implantable selon une des revendications précédentes, caractérisé en ce que le défibrillateur implantable comprend un boîtier (20) implantable à l'intérieur duquel sont disposés le circuit de charge (2), la capacité (3) et le dispositif de commutation (10, 11).

6. Système de défibrillateur implantable selon la revendication 5, caractérisé en ce que le dispositif de limitation du courant (19, 28, 40) est disposé dans le boîtier (20).

7. Système de défibrillateur implantable selon la revendication 5, caractérisé en ce que le système de défibrillateur implantable comprend une alimentation d'électrodes, dans laquelle le dispositif de limitation de courant est disposé.

8. Système de défibrillateur implantable selon la revendication 5, caractérisé en ce que le système de défibrillateur implantable comprend un module de raccordment (22) pourvu d'éléments de connexion (23) pour la liaison avec le boîtier du défibrillateur (25) et des éléments de connexion (26) supplémentaires pour la liaison avec au moins une des électrodes, dans lequel le dispositif (21) de limitation de courant est disposé.
